(19) Europäisches Patentamt
European Patent Office
Office européen des brevets

(11) **EP 3 386 560 B1**

(12) **EUROPÄISCHE PATENTSCHRIFT**

(45) Veröffentlichungstag und Bekanntmachung des Hinweises auf die Patenterteilung:
**05.04.2023 Patentblatt 2023/14**

(21) Anmeldenummer: **16806117.4**

(22) Anmeldetag: **06.12.2016**

(51) Internationale Patentklassifikation (IPC):
**A61M 1/14** *(2006.01)* **A61M 1/34** *(2006.01)*
**A61M 5/14** *(2006.01)*

(52) Gemeinsame Patentklassifikation (CPC):
**A61M 1/342; A61M 1/14; A61M 5/1684;**
A61M 5/14; A61M 2205/3331; A61M 2205/3389

(86) Internationale Anmeldenummer:
**PCT/EP2016/079935**

(87) Internationale Veröffentlichungsnummer:
**WO 2017/097774 (15.06.2017 Gazette 2017/24)**

(54) **BLUTBEHANDLUNGSVORRICHTUNG ZUM BESTIMMEN EINES PARAMETERS EINES FLÜSSIGEN MEDIKAMENTS WÄHREND EINER EXTRAKORPORALEN BLUTBEHANDLUNG**

BLOOD TREATMENT DEVICE FOR DETERMINING A PARAMETER OF A FLUID MEDICINE DURING AN EXTRACORPOREAL BLOOD TREATMENT

DISPOSITIF DE TRAITEMENT DU SANG POUR LA DÉTERMINATION D'UN PARAMÈTRE D'UN MÉDICAMENT LIQUIDE PENDANT UN TRAITEMENT EXTRACORPOREL DU SANG

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **08.12.2015 DE 102015121356**

(43) Veröffentlichungstag der Anmeldung:
**17.10.2018 Patentblatt 2018/42**

(73) Patentinhaber: **Fresenius Medical Care Deutschland GmbH**
**61352 Bad Homburg (DE)**

(72) Erfinder:
• **GAGEL, Alfred**
**96123 Litzendorf (DE)**
• **NÜRNBERGER, Thomas**
**97705 Burkardroth (DE)**
• **FISCHER, Karsten**
**97421 Schweinfurt (DE)**

(74) Vertreter: **Bobbert & Partner Patentanwälte PartmbB**
**Postfach 1252**
**85422 Erding (DE)**

(56) Entgegenhaltungen:
EP-A1- 2 319 553    DE-A1-102007 026 010
US-A1- 2005 145 009    US-A1- 2011 257 591
US-A1- 2012 150 141    US-A1- 2013 218 123
US-A1- 2014 039 447

**Beschreibung**

Die vorliegende Erfindung betrifft eine Blutbehandlungsvorrichtung gemäß Anspruch 1.

Manche Dialysepatienten müssen, wie andere Patienten auch, Medikamente einnehmen, die oral oder auch intravenös verabreicht werden können. Die Kenntnis der bereits verabreichten Menge an Medikament ist regelmäßig von Bedeutung.

Aus der EP 2 319 553 A1 ist ein Schlauchsatz mit einem Zugang zum Anschließen von Phiolen bekannt.

Systeme und Methoden, um eine Überdosierung von Medikamenten zu verhindern, sind in der US 2014/0039447 A1 offenbart.

Aus der US 2013/0218123 A1 sind Dorne für Medikamentenphiolen, Schlauchsätze und entsprechende Systeme bekannt.

In der US 2012/0150141 A1 sind eine Dialysemaschine, ein Verteiler hierfür sowie ein Arbeitsverfahren hierzu offenbart.

Aus der US 2005/0145009 A1 ist eine Erkennung von leeren Containern mittels behälterseitiger Druckmessung bekannt.

Eine Volumenüberwachung für implantierbare Fluidfördersysteme ist in der US 2011/0257591 A1 offenbart.

Aus der DE 10 2007 026010 A1 sind eine Vorrichtung zur Steuerung einer Einrichtung zum Fördern von Blut sowie ein Verfahren zum Fördern von Blut in einer Blutleitung eines extrakorporalen Blutkreislaufs einer extrakorporalen Blutbehandlungsvorrichtung bekannt.

Eine Aufgabe der vorliegenden Erfindung ist es, eine entsprechend eingerichtete Blutbehandlungsvorrichtung vorzuschlagen, mit welcher ein Verfahren zum Bestimmen eines Parameters für das Applizieren flüssiger Medikamenteausgeführt wird.

Offenbart wird somit ein Verfahren zum Bestimmen eines Parameters, welcher eine Aussage macht über die, insbesondere gerade ablaufende, Zugabe eines flüssigen Medikaments in ein Leitungsinneres eines extrakorporalen Kreislaufs für eine, insbesondere extrakorporale, Blutbehandlung.

Das Verfahren umfasst das Bereitstellen einer Blutbehandlungsvorrichtung mit einer Pumpe, beispielsweise einer Blutpumpe, verbunden mit einem extrakorporalen Kreislauf. Letzter weist optional wenigstens einen arteriellen Leitungsabschnitt und optional wenigstens einen venösen Leitungsabschnitt auf. Er weist ferner einen Medikamentenbehälter mit einem flüssigen Medikament auf, zudem eine Verbindungsvorrichtung zum Verbinden des Medikamentenbehälters mit dem extrakorporalen Kreislauf.

Die Verbindungsvorrichtung weist eine Einlassleitung und eine Auslassleitung auf, beide dienen zum Verbinden eines Inneren des Medikamentenbehälters mit dem Leitungsinneren des extrakorporalen Kreislaufs.

Die Einlassleitung ist mit einem ersten Abschnitt des extrakorporalen Kreislaufs in Fluidkommunikation verbunden. Die Auslassleitung ist mit einem zweiten Abschnitt des extrakorporalen Kreislaufs in Fluidkommunikation verbunden.

Das Verfahren umfasst schließlich das Verbinden der Einlassleitung mit einem ersten Abschnitt des extrakorporalen Kreislaufs und das Verbinden der Auslassleitung mit einem zweiten Abschnitt des extrakorporalen Kreislaufs.

Die erfindungsgemäße Blutbehandlungsvorrichtung weist wenigstens auf oder ist verbunden mit: einer Pumpe, z. B. einer Blutpumpe, einer Steuereinrichtung, einem extrakorporalen Kreislauf mit einem Leitungsinneren. Der extrakorporale Kreislauf ist vorbereitet oder bereitgestellt gemäß dem offenbarten Verfahren. Alternativ oder ergänzend weist der extrakorporale Kreislauf wenigstens einen arteriellen Leitungsabschnitt und wenigstens einen venösen Leitungsabschnitt auf. Der extrakorporale Kreislauf ist verbunden mit einer Verbindungsvorrichtung, welche zum Verbinden eines Medikamentenbehälters, welcher ein flüssiges Medikament aufweist, mit dem extrakorporalen Kreislauf dient. Die Verbindungsvorrichtung weist eine Einlassleitung und eine Auslassleitung auf, jeweils zum Verbinden eines Inneren des Medikamentenbehälters mit dem Leitungsinneren des extrakorporalen Kreislaufs. Die Einlassleitung ist mit einem ersten Abschnitt des extrakorporalen Kreislaufs in Fluidkommunikation verbunden; die Auslassleitung ist mit einem zweiten Abschnitt des extrakorporalen Kreislaufs in Fluidkommunikation verbunden. Der extrakorporale Kreislauf ist mit der Blutbehandlungsvorrichtung verbunden.

Die erfindungsgemäße Blutbehandlungsvorrichtung weist wenigstens ferner auf oder ist verbunden mit einem Drucksensor zum Ermitteln des Drucks im ersten oder zweiten Abschnitt.

Die Steuervorrichtung ist konfiguriert und/oder programmiert zum Bestimmen eines Parameters, welcher eine Menge oder ein Volumen eines bereits applizierten Medikaments und/oder eine zur vollständigen Verabreichung einer vorbestimmten Menge oder eines vorbestimmten Volumens des Medikaments erforderliche Restzeit ist.

Erfindungsgemäße Ausführungsformen können manche, einige oder alle der folgenden Merkmale in beliebiger Kombination aufweisen, soweit dies für den Fachmann nicht erkennbar technisch unmöglich ist. Insbesondere kann die Steuervorrichtung programmiert sein, um jeden einzelnen der hierin genannten Schritte zum Berechnen oder Ermitteln von Werten, insbesondere nach einer der hierin aufgeführten Gleichungen, durchzuführen, gleich, ob für sich allein oder in beliebiger Kombination von Schritten. Vorteilhafte Weiterbildungen der vorliegenden Erfindung sind jeweils auch Gegenstand der Unteransprüche.

Bei allen folgenden Ausführungen ist der Gebrauch des Ausdrucks "kann sein" bzw. "kann haben" usw. synonym zu "ist vorzugsweise" bzw. "hat vorzugsweise" usw. zu verstehen und soll erfindungsgemäße Ausführungsformen erläutern.

Wann immer hierin Zahlenworte genannt werden, so versteht der Fachmann diese als Angabe einer zahlenmäßig unteren Grenze. Sofern dies zu keinem für den Fachmann erkennbaren Widerspruch führt, liest der Fachmann daher beispielsweise bei der Angabe "ein" oder "einem" stets "wenigstens ein" oder "wenigstens einem" mit. Dieses Verständnis ist ebenso von der vorliegenden Erfindung mit umfasst wie die Auslegung, dass ein Zahlenwort wie beispielsweise "ein" alternativ als "genau ein" gemeint sein kann, wo immer dies für den Fachmann erkennbar technisch möglich ist. Beides ist von der vorliegenden Erfindung umfasst und gilt für alle hierin verwendeten Zahlenworte.

In einigen beispielhaften, erfindungsgemäßen Ausführungsformen weist die Einlassleitung ein erstes Rückschlagventil oder eine andere geeignete Strömungsbegrenzungseinrichtung auf, welche(s) einen Fluss durch die Einlassleitung nur in das Innere des Medikamentenbehälters oder in diesen hinein erlaubt, nicht aber durch die Einlassleitung aus dem Inneren heraus.

In manchen beispielhaften, erfindungsgemäßen Ausführungsformen weist die Auslassleitung ein zweites Rückschlagventil oder eine andere geeignete Strömungsbegrenzungseinrichtung auf, welche(s) einen Fluss über die Auslassleitung nur in Richtung zum Äußeren des Medikamentenbehälters oder aus diesem heraus erlaubt, nicht aber durch die Auslassleitung in das Innere hinein.

In einigen beispielhaften, erfindungsgemäßen Ausführungsformen fallen der erste Abschnitt und der zweite Abschnitt zusammen oder sind identisch, in anderen sind sie voneinander verschieden.

In einigen beispielhaften, erfindungsgemäßen Ausführungsformen umfasst das Verfahren das Verbinden des extrakorporalen Kreislaufs mit einer Blutbehandlungsvorrichtung und/oder mit einem Medikamentenbehälter. Die Blutbehandlungsvorrichtung und/oder der extrakorporale Kreislauf weisen wenigstens eine Blutpumpe zum Fördern von Blut innerhalb des Leitungsinneren auf. Sie kann ferner optional einen arteriellen Drucksensor, eine venöse Klemme, einen venösen Blasenfänger und/oder einen Kompressor aufweisen oder hiermit verbunden sein.

In einigen beispielhaften, erfindungsgemäßen Ausführungsformen sind der erste Abschnitt und der zweite Abschnitt des extrakorporalen Kreislaufs derart ausgewählt, dass der im ersten Abschnitt herrschende Druck in einem sich stets an das offenbarte Verfahren anschließenden, bestimmungsgemäßen Gebrauch des extrakorporalen Kreislaufs höher als im zweiten Abschnitt ist oder sein wird (dauerhaft oder zumindest vorübergehend, z. B. über die meiste Zeit des Gebrauchs).

In einigen beispielhaften, erfindungsgemäßen Ausführungsformen sind der erste Abschnitt und der zweite Abschnitt des extrakorporalen Kreislaufs derart ausgewählt, dass der im ersten Abschnitt herrschende Druck im bestimmungsgemäßen Gebrauch des extrakorporalen Kreislaufs so hoch wie im zweiten Abschnitt ist oder sein wird (dauerhaft oder zumindest vorübergehend, z. B. über die meiste Zeit des Gebrauchs).

In einigen beispielhaften, erfindungsgemäßen Ausführungsformen werden oder sind sowohl die Einlassleitung als auch die Auslassleitung mit einem Inneren eines venösen Blasenfängers des extrakorporalen Kreislaufs verbunden.

In einigen beispielhaften, erfindungsgemäßen Ausführungsformen ist die Steuervorrichtung ferner konfiguriert und/oder programmiert, um ein (hierin als Teilvolumen) bezeichnetes Volumen des flüssigen Medikaments zu ermitteln, welches während eines Pumpzyklus der Pumpe, insbesondere der Blutpumpe, über die Auslassleitung in den extrakorporalen Kreislauf abgegeben wurde. Ein Pumpzyklus kann bei einer optional als Rollenpumpe ausgestalteten Pumpe, insbesondere Blutpumpe, eine vollständige Rotation (d. h. um 360°) sein.

In einigen beispielhaften, erfindungsgemäßen Ausführungsformen ist die Steuervorrichtung ferner konfiguriert und/oder programmiert, um das aus dem Medikamentenbehälter über die Auslassleitung in den extrakorporalen Kreislauf abgegebene Volumen des Medikaments zu ermitteln. Dies kann durch Aufsummieren der in vorangegangenen oder in allen bisherigen Pumpzyklen der Pumpe, insbesondere Blutpumpe, aus dem Medikamentenbehälter in den extrakorporalen Kreislauf abgegebenen Teilvolumina erfolgen.

In manchen beispielhaften, erfindungsgemäßen Ausführungsformen ist die Steuervorrichtung ferner konfiguriert und/oder programmiert, um zu ermitteln, welches Volumen an Medikamenten noch aus dem Medikamentenbehälter über die Auslassleitung in den extrakorporalen Kreislauf abgegeben werden soll. Hierzu wird beispielsweise die Differenz zwischen dem bislang abgegebenen Volumen und einem, meist vorab und oft durch den Arzt, vorgegebenen Volumen ermittelt.

Optional kann als Parameter errechnet werden, welche Zeit noch vergehen wird bis, ceteris paribus, das vorgegebene oder abzugebende Volumen vollständig abgegeben sein wird. Diese Zeit, hierin auch als Restzeit bezeichnet, kann angezeigt werden. Eine geeignete Anzeigevorrichtung kann erfindungsgemäß vorgesehen sein. Die Steuervorrichtung kann zum Errechnen und/oder Anzeigen programmiert sein.

In einigen beispielhaften, erfindungsgemäßen Ausführungsformen ist die Steuervorrichtung ferner konfiguriert und/oder programmiert, um eine Zeitdauer zu ermitteln, während welcher das Rückschlagventil der Auslassleitung seit einem Referenzzeitpunkt geöffnet ist oder war. Der Referenzzeitpunkt kann der Zeitpunkt sein, zu welchem dieses Rückschlagventil geöffnet wurde.

Die Kenntnis der Öffnungsdauer dieses Rückschlagventils sowie der während der Öffnungsdauer jeweils vorliegenden Druckverläufe sowohl des Drucks im Medikamentenbehälter als auch im ersten oder zweiten Abschnitt, etwa im Blasenfänger des Kreislaufs, kann erlauben, das über die Auslassleitung applizierte Teilvolumen auf einfachste Weise zu berechnen.

Die Öffnungsdauer (im Folgenden auch als tour bezeichnet, der Öffnungszeit des Ausgangsventils (passiv, da Rückschlagventil) kann aus Druckverläufen berechnet oder mittels einer Einrichtung gemessen werden.

In einigen beispielhaften, erfindungsgemäßen Ausführungsformen ist die Steuervorrichtung ferner konfiguriert und/oder programmiert, zum Ermitteln des im ersten und/oder zweiten Abschnitt herrschenden Drucks, und/oder dessen Verlaufs, während wenigstens eines Pumpzyklus der Pumpe, insbesondere der Blutpumpe, oder während eines vorbestimmten Zeitraums, etwa der Öffnungsdauer des Rückschlagventils der Auslassleitung.

Der dabei als maximaler Wert im ersten Abschnitt erkannte Druckwert kann optional zumindest bis zu seiner Verwendung in weitergehenden Berechnungen gespeichert werden. Er kann vorteilhaft als Eingangsgröße bei der Berechnung des im Medikamentenbehälter zum Zeitpunkt des Schließens des Einlassrückschlagventils herrschenden Drucks und damit des zu Beginn der sich anschließenden Ausbringphase im Medikamentenbehälter herrschenden Drucks verwendet werden, beim Berechnen einer Compliance des Medikamentenbehälters und beim Berechnen einer Zeitkonstante (wie sie zur Gleichung 2.11 weiter unten als $\tau$ definiert ist), mit welcher das Medikament aus dem Medikamentenbehälter strömt.

In einigen beispielhaften, erfindungsgemäßen Ausführungsformen ist die Steuervorrichtung ferner konfiguriert und/oder programmiert, um einen im Medikamentenbehälter herrschenden Druck zu berechnen.

In einigen beispielhaften, erfindungsgemäßen Ausführungsformen ist die Steuervorrichtung ferner konfiguriert und/oder programmiert, um mittels einer Fördereinrichtung, etwa eines Kompressors der Blutbehandlungsvorrichtung, Luft in das Innere des extrakorporalen Kreislaufs einzubringen, insbesondere in den venösen Blasenfänger. Dies kann über die Einlassleitung zur Erhöhung des Drucks im Medikamentenbehälter führen und ein Austreiben des Medikaments über die Auslassleitung begünstigen. Hierzu bietet es sich an, die Einlassleitung mit dem entsprechenden Abschnitt, z. B. dem venösen Blasenfänger, verbunden zu haben.

In einigen beispielhaften, erfindungsgemäßen Ausführungsformen ist die Blutbehandlungsvorrichtung eine Hämodialysevorrichtung, eine Hämofiltrationsvorrichtung oder eine Hämodiafiltrationsvorrichtung.

Der extrakorporale Kreislauf ist in bestimmten Ausführungsformen der vorliegenden Erfindung ein Schlauchset. In jedem Fall ist der extrakorporale Kreislauf vorgesehen zum extrakorporalen Leiten von Blut eines Patienten, beispielsweise bei der Hämodialyse, der Hämofiltration, der Hämodiafiltration oder dergleichen.

In einigen erfindungsgemäßen Ausführungsformen ist der extrakorporale Kreislauf zumindest abschnittsweise als integraler und ggf. unlösbarer Bestand einer Blutkassette ausgestaltet, in anderen nicht. So kann sich ein frei beweglicher Schlauchabschnitt des extrakorporalen Kreislauf in einem einstückig oder integral auf oder in der Blutkassette, hergestellten Schlauchabschnitt fortsetzen und umgekehrt.

Beispielhafte Ausführungsformen einer Blutkassette sind insbesondere in der Anmeldung der Anmelderin mit der Veröffentlichungsnummer DE 10 2009 018 664 A1 mit dem Titel *"Externe Funktionseinrichtung, Blutbehandlungsvorrichtung zum Aufnehmen einer erfindungsgemäßen externen Funktionseinrichtung, sowie Verfahren"*, die am 23.04.2009 beim Deutschen Patent- und Markenamt eingereicht wurde, und in der Anmeldung der Anmelderin mit der Veröffentlichungsnummer DE 10 2009 024 468 A1 mit demselben Titel, die am 10.06.2009 beim Deutschen Patent- und Markenamt eingereicht wurde, offenbart.

Der optionale arterielle Leitungsabschnitt des extrakorporalen Kreislaufs ist in bestimmten Ausführungsformen der vorliegenden Erfindung jener Leitungsabschnitt, in welchen das den Patientenkörper zum Zwecke der extrakorporalen Blutbehandlung verlassende Patientenblut einströmt und in welchem es sich vor Eintritt in die Blutbehandlungseinrichtung, z. B. einen Dialysator, befindet.

In bestimmten Ausführungsformen der vorliegenden Erfindung ist oder umfasst der Kreislauf den arteriellen Nadelanschluss zum Patienten, z. B. den arteriellen Nadelanschluss für ein Double-Needle-Dialyseverfahren.

Der optionale venöse Leitungsabschnitt des extrakorporalen Kreislaufs ist in manchen Ausführungsformen der vorliegenden Erfindung jener Leitungsabschnitt, aus welchem das extrakorporal behandelte Patientenblut nach seiner Behandlung in einer Blutbehandlungseinrichtung, beispielsweise einem Dialysator, zum Patientenkörper hin oder in diesen zurückströmt.

In bestimmten Ausführungsformen der vorliegenden Erfindung ist der erste Abschnitt Teil der Hochdruckseite des extrakorporalen Kreislaufs, der zweite Abschnitt Teil der Niederdruckseite.

In einigen erfindungsgemäßen, beispielhaften Ausführungsformen ist der erste Abschnitt stromab (Hochdruckseite) einer Pumpe angeordnet, der zweite Abschnitt hingegen stromauf (Niederdruckseite) dieser Pumpe. Die Pumpe kann insbesondere die Blutpumpe, die optionale Substituatpumpe oder die optionale Single-Needle-Pumpe sein.

In manchen erfindungsgemäßen, beispielhaften Ausführungsformen ist der erste Abschnitt stromauf des Dialysators angeordnet, der zweite Abschnitt hingegen stromab des Dialysators. Der Druckabfall kann hierbei als $\Delta P = Q_b$

* $R_b$ mit $Q_b$ als Blutfluss und $R_b$ als Dialysator-Widerstand ermittelt werden.

In einigen erfindungsgemäßen, beispielhaften Ausführungsformen ist der erste Abschnitt in einer Safeline und/oder stromab der Substituatpumpe angeordnet, der zweite Abschnitt hingegen im venösen Blasenfänger. Die Druckdifferenz kann optional durch den Öffnungsdruck eines Rückschlagventils realisiert werden.

Der Volumenstrom $Q_{Med}$ des flüssigen Medikaments kann wie folgt berechnet werden:

$$Q_{Med} = \frac{P_+ - P_-}{R_{IN} + R_{OUT}} \qquad (1.1)$$

Dabei steht

$P_+$      für den Druck auf der Hochdruckseite, also im ersten Abschnitt;

$P_-$      für den Druck auf der Niederdruckseite, also im zweiten Abschnitt;

$R_{IN}$      für den Strömungswiderstand in der Einlassleitung; und

$R_{OUT}$      für den Druck in der Auslassleitung.

Mit dem Ergebnis der Gleichung (1.1) können gemäß

Gleichung (1.2) das applizierte Volumen $V_{Med}$ oder die notwendige Zeitdauer (von 0 bis t), die erforderlich ist, um das Volumen zu applizieren, berechnet werden. Das Volumen $V_{Med}$ entspricht der gesamten Medikamentenmenge $V_{Med}$, die es (z. B. aufgrund einer Verschreibung durch den Arzt) zu verabreichen gilt.

$$V_{Med} = \int_0^t Q_{Med} * dt = \frac{\int_0^t (P_+ - P_-) * dt}{R_{IN} + R_{OUT}} \qquad (1.2)$$

In manchen Ausführungsformen der vorliegenden Erfindung werden die Förderraten der Pumpe, insbesondere der Blutpumpe, und/oder der zweiten Fördereinrichtung während des Applizierens des Medikaments aus dem Medikamentenbehälter mittels Drucküberwachung und/oder Druckmessung und/oder Druckbegrenzung überwacht und/oder geregelt.

In manchen Ausführungsformen der vorliegenden Erfindung wird der aktuelle Förderdruck mittels des venösen Drucksensors gemessen.

Die hierin genannte Steuereinrichtung ist in einigen erfindungsgemäßen Ausführungsformen als Regeleinrichtung ausgestaltet.

In manchen Ausführungsformen der vorliegenden Erfindung wird der im Vial herrschende Druck bei Öffnen des Rückschlagventils berechnet oder ermittelt.

In manchen Ausführungsformen der vorliegenden Erfindung wird die im Vial vorliegenden Luftmasse nach Öffnen des Rückschlagventils berechnet oder ermittelt.

In manchen Ausführungsformen der vorliegenden Erfindung wird das applizierte Teilvolumen $\Delta V_{Med}$ berechnet oder ermittelt.

Erfindungsgemäß wird der im Vial herrschende Druck mittels Modells berechnet oder ermittelt.

In manchen Ausführungsformen der vorliegenden Erfindung wird der maximale Druck im Blasenfänger $P_{BF,max}$ berechnet oder ermittelt.

In manchen Ausführungsformen der vorliegenden Erfindung erfolgt die Berechnung ausgehend vom maximalen Druck im Blasenfänger.

In manchen Ausführungsformen der vorliegenden Erfindung wird der zeitliche Druckverlauf im Vial berechnet oder ermittelt.

In manchen Ausführungsformen der vorliegenden Erfindung wird das applizierte Teilvolumen berechnet oder ermittelt.

In manchen Ausführungsformen der vorliegenden Erfindung wird das bislang applizierte Volumen berechnet oder ermittelt.

In manchen Ausführungsformen der vorliegenden Erfindung wird die verbleibende Applikationsdauer berechnet oder ermittelt.

Manche oder alle erfindungsgemäßen Ausführungsformen können einen, mehrere oder alle der oben und/oder im Folgenden genannten Vorteile aufweisen.

Die vorliegende Erfindung stellt ein einfaches und wenig aufwendiges Verfahren zum Verabreichen eines flüssigen Medikaments oder eines Medikaments in Lösung.

Manche Dialysepatienten müssen, wie andere Patienten auch, Medikamente einnehmen, die oral oder auch intravenös verabreicht werden können.

Dialysepatienten unterziehen sich, anders als andere Patienten, regelmäßig extrakorporalen Blutbehandlungen. Dabei wird Blut des Patienten diesem entnommen und mittels Blutpumpe durch den extrakorporalen Kreislauf gefördert und nach Durchlaufen beispielsweise eines Dialysators dem Patienten rückinfundiert.

Die vorliegenden Erfindung schlägt vorteilhafterweise vor, flüssige oder in Wasser gelöste Medikamente, die intravenös verabreicht werden können oder müssen, während des extrakorporalen Blutbehandlungsverfahrens zu applizieren, da der unmittelbare Zugang zum Blut bereits existiert und der Patient nicht zusätzlich punktiert werden muss. Außerdem wird dabei vorteilhaft sichergestellt, dass der Patient die Medikation zuverlässig erhält, d. h. die Verabreichung ist unabhängig von der Compliance des Patienten.

Erfindungsgemäß erfolgt die Dosierung und die Zugabe der Medikation mittels einer Pumpe, z. B. der Blutpumpe, welche zur Dialysebehandlung ohnehin in Gebrauch ist. Erfindungsgemäß bedarf es vorteilhafterweise keiner zusätzlichen (Medikamenten-)Pumpe. Das Pflegepersonal muss die Medikamente auch nicht etwa manuell über eine Spritze verabreichen. Damit können vorteilhafterweise zusätzliche geräteseitige Pumpen eingespart, die Kosten und die Komplexität des Systems niedrig gehalten werden.

Das erfindungsgemäß zu applizierende Medikament ist in einigen Ausführungsformen ein Eisenpräparat.

In einigen Ausführungsformen ist das erfindungsgemäß zu applizierende Medikament kein Antikoagulans, insbesondere enthält das Medikament vorzugsweise kein Heparin und/oder kein Zitrat.

Der erfindungsgemäße Medikamentenbehälter ist vorzugsweise aus einem starren, nicht elastischen Material gefertigt. Der erfindungsgemäße Medikamentenbehälter ist vorzugsweise keine Spritze, ist kein Teil einer Spritze und/oder umfasst keine Spritze.

In einigen Ausführungsformen ist der erfindungsgemäße Medikamentenbehälter nicht Teil einer Spritzenpumpe und/oder ist nicht mit einer Spritzenpumpe verbunden.

In einigen Ausführungsformen umfasst die erfindungsgemäße Blutbehandlungsvorrichtung keine Infusionspumpe, keine Spritzenpumpe und/oder ist mit keiner Spritzenpumpe und/oder mit keiner Infusionspumpe verbunden.

Während des Verfahrens wird das umschlossene Volumen des Medikamentenbehälters vorzugsweise nicht verändert, insbesondere nicht verringert. Die erfindungsgemäße Blutbehandlungsvorrichtung umfasst vorzugsweise keine Einrichtung, die konfiguriert ist, um das umschlossene Volumen des Medikamentenbehälters zu verringern, insbesondere diesen zu komprimieren.

Vorteilhafterweise kann auch ein andernfalls erforderliches Synchronisieren einer externen Medikamentenpumpe oder Infusionspumpe mit der Blutbehandlungsvorrichtung entfallen. So muss, erfolgt die Dosierung mittels der Blutpumpe als Pumpe, beim Stopp der Blutpumpe durch den Anwender oder durch einen Alarm keine Medikamentenpumpe eigens angehalten werden.

Von Vorteil ist ferner, dass durch Wegfall eines manuellen Verabreichens des Medikaments, was erfindungsgemäß möglich ist, Arbeitskraft eingespart werden kann. Arbeitskraft bzw. Personal ist insbesondere erforderlich, wenn Medikamente in mehreren Einzeldosen gegeben werden müssen, wie dies etwa bei Eisenpräparaten der Fall ist.

Durch die erfindungsgemäß mögliche Automatisierung wird außerdem menschliches Fehlerverhalten (wie Vergessen von Teilinfusionen oder der Gesamtinfusion) ausgeschlossen.

Diese Vorteile sind durch das Dosieren oder Zugeben des flüssigen Medikaments mittels der Pumpe oder der Blutpumpe der Blutbehandlungsvorrichtung wie hierin beschrieben erzielbar.

Erfindungsgemäß kann ferner auf einfache Weise die Förderrate des Medikaments bestimmt werden. Dadurch kann zum einen sicher festgelegt werden, wann das Volumen vollständig verabreicht worden ist. Zum anderen kann bei einer zu kleinen oder bei einer zu hohen Förderrate auf die Behandlung eingewirkt werden und die Förderrate wieder in einen akzeptablen Bereich verschoben werden. Dies ist mithilfe der Erfindung zudem möglich, ohne hierfür zusätzliche Aktoren wie Aktor, Stößel, Medikamentenpumpe oder Luftkompressor vorsehen zu müssen.

Alle mit dem offenbarten Verfahren erzielbaren Vorteile lassen sich in bestimmten erfindungsgemäßen Ausführungsformen ungeschmälert auch mit den erfindungsgemäßen Vorrichtungen erzielen.

Im Folgenden wird die vorliegende Erfindung unter Bezugnahme auf die beigefügten Figuren rein exemplarisch beschrieben. Es gilt:

**Fig. 1**    zeigt in vereinfachter Darstellung eine erfindungsgemäße Blutbehandlungsvorrichtung;

**Fig. 2**    zeigt Druck- und Volumenverläufe über der Zeit, welche beim Ausführen einer beispielhaften Ausführungsform des offenbarten Verfahrens mittels der in Fig. 1 gezeigten Anordnung auftreten oder gemessen werden können; und

**Fig. 3**    zeigt Druck- und Volumenverläufe über der Zeit, welche beim Ausführen einer beispielhaften Ausführungsform des offenbarten Verfahrens mittels der in Fig. 1 gezeigten Anordnung auftreten oder gemessen werden können.

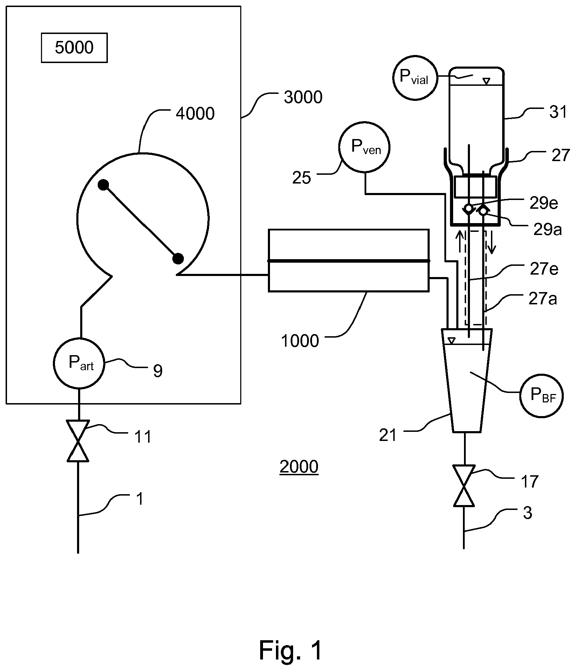

**Fig. 1** zeigt schematisch vereinfacht eine erfindungsgemäße Blutbehandlungsvorrichtung 3000, an welche eine Funktionseinrichtung 1000 und ein hiermit verbundener extrakorporaler Kreislauf 2000 in Fluidverbindung angeschlossen sind. Die Blutbehandlungsvorrichtung 3000 ist ferner rein optional mit einer Blutkassette verbunden.

Die Blutkassette ist in einigen erfindungsgemäßen Ausführungsformen als Disposable oder als Wegwerfartikel ausgestaltet.

Die Funktionseinrichtung 1000 ist z. B. ein Blutfilter oder ein Dialysator.

Die in Fig. 1 nur durch einige ihrer Einrichtungen repräsentierte Blutbehandlungsvorrichtung 3000 ist ein Dialysegerät. Mit ihr kann das hier beschriebene Verfahren ablaufen, optional ganz oder im Wesentlichen automatisiert. Die Blutbehandlungsvorrichtung 3000 weist eine Blutpumpe 4000 auf. Sie weist optional eine nicht gezeigte zweite Fördereinrichtung auf. Die folgende Erläuterung nimmt Bezug auf die Blutpumpe 4000. Diese könnte alternativ eine Pumpe sein, welche keine Blutpumpe ist, etwa eine Substituatpumpe.

Der extrakorporale Kreislauf 2000 weist hier rein optional einen arteriellen Leitungsabschnitt 1 sowie einen venösen Leitungsabschnitt 3 auf. Es können andere Leitungsabschnitte vorgesehen sein.

Der arterielle Leitungsabschnitt 1 weist optional einen arteriellen Nadelanschluss (nicht gezeigt) auf.

Der arterielle Leitungsabschnitt 1 weist einen arteriellen Drucksensor auf, welcher an der mit Bezugszeichen 9 bezeichneten Stelle an den Kreislauf 2000 gekoppelt ist ohne notwendigerweise selbst Bestandteil des Kreislaufs 2000 zu sein.

Der arterielle Leitungsabschnitt 1 weist eine arterielle Klemme 11 auf.

Der venöse Leitungsabschnitt 3 weist optional einen nicht gezeigten venösen Luftblasendetektor/optischen Sensor auf.

Der venöse Leitungsabschnitt 3 weist eine venöse Klemme 17 auf.

Stromab der Funktionseinrichtung 1000 ist ein venöser Blasenfänger 21 vorgesehen.

Der im mit Flüssigkeit befüllten Abschnitt des Blasenfängers 21 herrschende Druck wird hierin als $P_{BF}$ bezeichnet.

Ein venöser Drucksensor trägt das Bezugszeichen 25. Er kann beispielsweise angeordnet sein, um den in einem oberen Abschnitt des Blasenfängers 21 herrschenden Druck $P_{Ven}$ zu messen.

Eine Verbindungsvorrichtung 27 ist zur Aufnahme eines Medikamentenbehälters vorgesehen und weist Leitungen zum Herstellen einer Fluidverbindung zwischen dem Medikamentenbehälter und dem Leitungsinneren des extrakorporalen Kreislaufs 2000 auf.

Die Verbindungsvorrichtung 27 weist in Fig. 1 exemplarisch einen doppellumigen Tubus oder doppellumigen Verbinder auf, welcher in Fig. 1 mittels Strichlinie angedeutet ist. Der doppellumige Verbinder weist im Beispiel der Fig. 1 zwei Leitungen 27e und 27a zum Leiten von Fluid auf.

Die Leitung 27a ist im Beispiel der Fig. 1 mit einem Rückschlagventil 29a verbunden, die Leitung 27e ist mit einem Rückschlagventil 29e verbunden.

Die Leitungen 27a und 27e sind mit dem Inneren des Vial 31 als Beispiel eines Medikamentenbehälters oder mit einem andersartigen Behälter verbunden, welcher ein flüssiges Medikament aufweist oder aufweisen kann. Der im Vial 31 herrschende Druck wird hierin als $P_{Vial}$ bezeichnet.

Das Rückschlagventil 29e erlaubt einen Fluss innerhalb der Leitung 27e vom Blasenfänger 21 in Richtung zum Vial 31, ausreichenden Fluiddruck zum Öffnen des Rückschlagventils 29e vorausgesetzt, nicht jedoch in umgekehrter Richtung. Das Rückschlagventil 29e wird daher hierin als Einlassrückschlagventil 29e bezeichnet. Die Leitung 27e erlaubt ein Einbringen von Fluid (kann hier stets Gas, insbesondere Luft, oder eine Flüssigkeit sein) in das Vial 31 und wird daher hierin auch als Einlassleitung 27e bezeichnet.

Das Rückschlagventil 29a erlaubt einen Fluss innerhalb der Leitung 27a vom Vial 31 in Richtung zur Blasenkammer 21, ausreichenden Fluiddruck zum Öffnen des Rückschlagventils 29a vorausgesetzt, nicht jedoch in umgekehrter Richtung. Das Rückschlagventil 29a wird daher hierin als Auslassrückschlagventil 29a bezeichnet. Die Leitung 27a erlaubt ein Ausbringen von Fluid aus dem Vial 31 und wird daher hierin auch als Auslassleitung 27a bezeichnet.

Im Beispiel der Fig. 1 ist die Einlassleitung 27e angeordnet, um im oberen, flüssigkeitsfreien Abschnitt des Blasenfängers 21 zu münden. Die Auslassleitung 27a ist angeordnet, um im mit Flüssigkeit befüllten Abschnitt des Blasenfängers 21 zu münden, also an Stellen identischen Drucks.

Im Beispiel der Fig. 1 sind die Einlass- und Auslassleitungen 27e bzw. 27a an der gleichen Stelle mit dem Kreislauf 2000 verbunden, so dass keine oder keine relevanten Druckdifferenzen bestehen. Der erste Abschnitt und der zweite Abschnitt fallen im Beispiel der Fig. 1 daher zusammen.

Bei dieser Anordnung kann die zeitliche und idealerweise periodische Änderung des Drucks an dieser Stelle ausgenutzt werden. In der Dialyse werden als Blutpumpen 4000 üblicherweise Schlauchrollenpumpen verwendet, wie auch in Fig. 1. Sie erzeugen mit jeder Umdrehung periodische Druckschwankungen. Wenn die vorwärtstreibende Rolle abhebt und die Okklusion des Schlauchs freigibt, entsteht ein Einbruch im Drucksignal, der anschließend wieder aufgebaut wird.

Dieses Vorgehen mittels des offenbarten Verfahrens in einer exemplarischen Ausführungsform wird im Folgenden genauer beschrieben.

Der Überdruck im Vial 31 kann durch periodisches kurzes Schließen der venösen Klemme 17 erzeugt oder verstärkt werden. Die Förderrate der Blutpumpe 4000 wird dabei beibehalten. Nach Erreichen eines venösen Druckwerts, der kleiner als der obere venöse Skalenendwert einer Skala für den venösen Druckwert sein muss, wird anschließend die venöse Klemme 17 geöffnet. Durch die hierbei einstellbaren Druckdifferenzen, kann ein Medikament mit relativ hoher Dosierrate (beispielsweise als sogenannte Bolusapplikation) verabreicht werden.

Die Druckerhöhung im Vial 31 kann alternativ oder ergänzend auch durch die Zudosierung von externer Luft in das Schlauchsystem erzeugt werden. Sinnvollerweise wird hier der nicht dargestellte Kompressor verwendet, der auch zum Senken des Flüssigkeitspegels oder -spiegels im venösen Blasenfänger 21 verwendet wird.

Die Ein- und Auslassleitungen 27a, 27e des Vials 31 werden hierzu an den venösen Blasenfänger 21 angeschlossen. Er besitzt ein - auch geräteseitig automatisch - einstellbares Luftvolumen. Er befindet sich stromab der Blutpumpe 4000. Durch das Abheben der Rollen entstehen periodische Druckschwankungen, die zum Entleeren des Vials 31 verwendet werden.

Die ausreichend genaue Dosierung des Medikaments oder Entleerung des Vials 31 kann gemäß folgender Voraussetzung und Annahmen ermittelt werden:

Der im venösen Blasenfänger 21 herrschende Druck $P_{BF}$ wird ausschließlich von den Behandlungsparametern (Blutfluss $Q_b$, Blutviskosität $\eta$, Durchmesser der venösen Nadel) und der Ausgestaltung von Komponenten und/oder Einstellungen der Blutbehandlungsvorrichtung bestimmt. Ob das Vial 31 eingesetzt ist oder nicht, spielt keine Rolle für den Druck $P_{BF}$, denn die Volumenströme ins Vial 31 oder aus dem Vial 31 heraus sind sehr klein gegenüber dem Blutfluss $Q_b$.

In der Füllphase strömt Luft in das Vial 31 ein. Hierzu muss das Einlassrückschlagventil 29e öffnen.

Das Einlassrückschlagventil 29e öffnet, sobald die Druckdifferenz zwischen Blasenfänger 21 und Vial 31 größer ist als der Öffnungsdruck des Einlassrückschlagventils 29e.

Da Luft eine sehr niedrige Viskosität aufweist und die Einlassleitung einen relativ großen Durchmesser besitzt, ist der Strömungswiderstand der Einlassseite sehr niedrig. Daher wird hier davon ausgegangen, dass sich der Druck im Vial 31, $P_{Vial}$, sofort mit dem Druck $P_{BF}$ im Blasenfänger 21 ausgleicht, wenn das Rückschlagventil 29a öffnet, d. h.

$$P_{Vial} = P_{BF} - \Delta P_{IN} \qquad (2.1)$$

mit

$\Delta P_{IN}$      Öffnungsdruck des Rückschlagventil 29a

Das Luftvolumen $V_L$ im Vial 31 verändert sich dadurch nicht, die Volumenausdehnung des Gefäßes ist vernachlässigbar und das Auslassrückschlagventil 29a der Auslassleitung 27a ist bei diesen Druckverhältnissen sicher geschlossen, so dass kein Flüssigkeitsvolumen entweichen kann.

Die Luftmasse $m_L$ im Vial 31 erhöht sich allerdings und berechnet sich nach der allgemeinen Gasgleichung ($V_L$, T = konst.):

$$m_L = \frac{P_{Vial} * V_L}{R_L * T} \qquad (2.2)$$

mit

$m_L$      Luftmasse im Vial [g]
$V_L$      Luftvolumen im Vial [mL]
$R_L$      spezifische Gaskonstante für Luft [287 J/kg/K]
T      absolute Temperatur [K]

Die eingeschlossene Luft ist kompressibel und bildet dadurch die Compliance C:

$$C = -\frac{dV}{dP} = \frac{V_L}{P_{Vial}} \qquad (2.3)$$

Während einer Druckperiode kann die Compliance C näherungsweise als konstant betrachtet werden, weil nach dem Schließen des Einlassrückschlagventils 29a die Luftmasse $m_L$ konstant bleibt, und die Druckänderungen

$\Delta P_{Vial}$ im Vial 31 während der Ausströmphase klein sind im Vergleich zum absoluten Druck $P_{Vial}$ im Vial 31, da immer im Überdruckbereich gearbeitet wird.

Wenn das Einlassrückschlagventil 29e in der Einlassleitung 27e geöffnet ist und der Druck $P_{BF}$ im Blasenfänger 21 weiter ansteigt, so steigt gemäß Gleichung (2.1) auch der Druck $P_{Vial}$ im Vial 31. Wenn der Druck $P_{BF}$ im Blasenfänger 21 fällt, schließt das Rückschlagventil 29e und der Druck $P_{Vial}$, der im Vial 31 eingeschlossen wird, ergibt sich zu:

$$P_{Vial,in} = P_{BF,max} - \Delta P_{IN} \qquad (2.4)$$

mit

$P_{BF,max}$      Peak- oder Spitzenwert des Drucks im Blasenfänger oder Druck, der im Blasenfänger 21 erreicht wird, unmittelbar bevor das Rückschlagventil 29e schließt.

Die Ausströmphase ist dadurch gekennzeichnet, dass das Rückschlagventil 29a öffnet oder geöffnet ist und das flüssige Medikament aus dem Vial 31 strömt.

Das Rückschlagventil 29a öffnet, sobald die Druckdifferenz zwischen Vial 31 und Blasenfänger 21 größer ist als der Öffnungsdruck $\Delta P_{OUT}$ des Rückschlagventils 29a. Der Volumenstrom $Q_{Med}$ ergibt sich zu:

$$Q_{Med} = \frac{P_{Vial} - P_{BF}}{R_{OUT}} \qquad (2.5)$$

Damit kann das applizierte Teilvolumen $\Delta V_{Med}$ berechnet werden:

$$\Delta V_{Med} = \int_0^{t_{OUT}} Q_{Med} * dt = \frac{1}{R_{OUT}} * \int_0^{t_{OUT}} (P_{Vial} - P_{BF}) * dt \qquad (2.6)$$

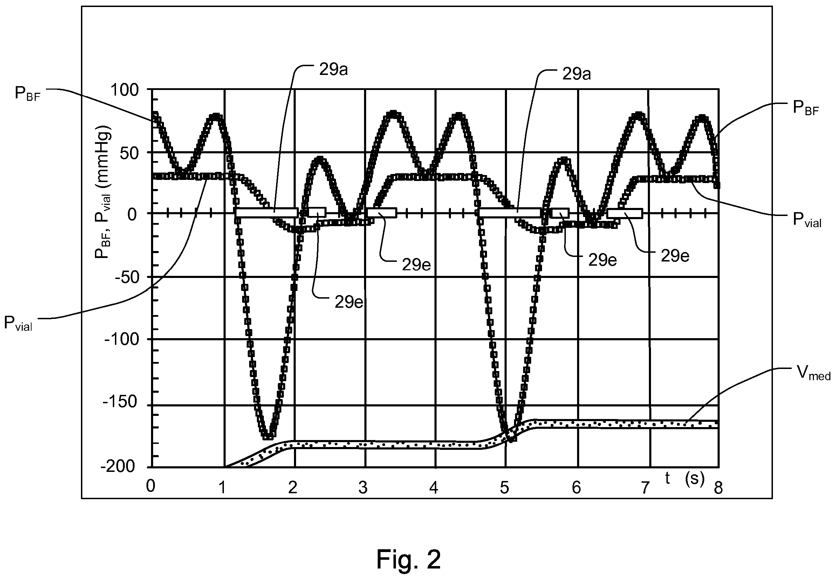

Die Zeit $t_{OUT}$ entspricht der Dauer, in der das Rückschlagventil 29a geöffnet ist. Es ist offen, solange die Druckdifferenz zwischen Vial 31 und dem Blasenfänger 21 größer ist als der Öffnungsdruck des Rückschlagventils 29a, siehe auch Fig. 2.

Während der Druck $P_{BF}$ im Blasenfänger 21 mit Hilfe des Drucksensors 25 für den venösen Rücklaufdruck gemessen werden kann, gibt es in der Regel keinen Drucksensor, um den Druck $P_{Vial}$ im Vial 31 messen zu können. Der Druck $P_{Vial}$ kann mithilfe eines Modells berechnet werden.

Das ausströmende Medikament schafft Raum im Vial 31, in den sich das Luftvolumen $V_L$ ausdehnen kann. Es gilt daher:

$$Q_{Med} = \frac{dV_L}{dt} \qquad (2.7)$$

Die Volumenausdehnung der Luft $V_L$ führt zu einer Druckentspannung im Vial 31. Die zeitliche Änderung folgt aus der allgemeinen Gasgleichung $P_{Vial} * V_L = m_L * R_L * T$ mit ($m_L$, T = konst.):

$$\frac{dP_{Vial}}{dt} * V_L + P_{Vial} * \frac{dV_L}{dt} = 0$$

Das Einsetzen von Gleichung (2.3) und Gleichung (2.7) in die vorstehende Gleichung ergibt:

$$\frac{dP_{Vial}}{dt} * C + Q_{Med} = 0$$

Mit Gleichung (2.5) folgt:    $\frac{dP_{Vial}}{dt} * C + \frac{P_{Vial} - P_{BF}}{R_{OUT}} = 0$    und schließlich:

$$\tau * \frac{dP_{vial}}{dt} + P_{Vial} = P_{BF}(t) \qquad (2.8)$$

wobei

$$\tau = C * R_{OUT} \qquad (2.9)$$

Die Lösung von Gleichung (2.8) liefert den zeitlichen Verlauf für den Druck im Vial 31 während der Ausströmphase:

$$P_{Vial}(t) = P_{Vial}(0) * e^{-t/\tau} + \frac{1}{t} * \int_0^t P_{BF}(\delta) * e^{-(t-\delta)/\tau} * d\delta \qquad (2.10)$$

mit

$P_{Vial(0)}$      Druck im Vial 31 zum Öffnungszeitpunkt des Rückschlagventils 29a. Er ergibt sich aus dem Druck, der am Ende der Füllphase im Vial 31 (siehe Gleichung (2.4)) eingeschlossen wird:

$$P_{Vial}(0) = P_{Vial,in} = P_{BF,max} - \Delta P_{IN} \qquad (2.11)$$

mit:

$\tau$      Zeitkonstante, mit der die Flüssigkeit aus dem Vial 31 strömt. Während einer Ausströmphase wird sie als Konstante betrachtet. Am Ende der Ausströmphase hat sich das Luftvolumen $V_L$ vergrößert. Am Ende der nächsten Füllphase erhöhen sich die Luftmasse $m_L$ und der Luftdruck $P_{Vial}$ im Vial 31. Jetzt muss nach Gleichung (2.3) die Compliance C erneut berechnet werden. Aus Gleichung (2.9) ergibt sich die neue Zeitkonstante $\tau$ für die nächste Ausströmphase in Gleichung (2.10).

Bei relativ großen Druckänderungen kann der mittlere Druck im Vial 31 zur genaueren Berechnung der Compliance C nach Gleichung (2.3) verwendet werden:

$$\overline{P}_{Vial} = \frac{1}{t_{OUT}} * \int_0^{t_{OUT}} P_{Vial}(t) * dt \qquad (2.12)$$

Mit Hilfe von Gleichung (2.6) kann das applizierte Teilvolumen $\Delta V_{Med}$ in dieser Ausströmphase berechnet werden. Das Aufsummieren aller applizierten Teilvolumina $\Delta V_{Med}$ ergibt das bisher insgesamt verabreichte Volumen $V_{Med}$.

Der mittlere Medikamentenfluss $\overline{Q}_{Med}$ während einer Periode T kann berechnet werden mit:

$$\overline{Q}_{Med} = \frac{\Delta V_{Med}}{T} \qquad (2.13)$$

Bei einem vorgegebenen Medikamentenvolumen $V_{Med,0}$, das verabreicht werden soll, kann mit Hilfe von Gleichung (2.13) die Dauer $t_{ges}$ für die vollständige Infusion berechnet werden:

$$t_{ges} = \frac{V_{Med,0}}{\overline{Q}_{Med}} \qquad\qquad (2.14)$$

Wenn die Rate $\overline{Q}_{Med}$, mit der das Medikament verabreicht wird, zu groß ist, bzw. wenn die Applikationsdauer $t_{ges}$ zu kurz ist, kann der Blutfluss $Q_b$ für eine gewisse Zeitdauer reduziert werden. Kleinere Blutflüsse haben kleinere Druckänderungen im venösen Blasenfänger zur Folge, wodurch die mittlere Rate $Q_{Med}$ sinkt. Der Blutfluss kann über mehrere Zyklen adaptiert werden.

Andererseits kann die Blutflussrate $Q_b$ erhöht werden, wenn die Rate $\overline{Q}_{Med}$, mit der das Medikament verabreicht wird, zu niedrig ist, bzw. wenn die Applikationsdauer $t_{ges}$ zu lang ist. Alternativ kann auch durch kurzzeitiges Schließen der venösen Absperrklemme oder Klemme 17 eine Erhöhung in den Druckspitzen realisiert werden.

Ein möglicher Ablauf des Verfahrens kann wie folgt aussehen:

Zunächst müssen Werte für folgende Parameter in Erfahrung gebracht oder eingestellt werden, die in folgender Tabelle 1 mit typischen Werten angegeben sind.

**Tabelle 1**

| Luft-Volumen im Vial 31 | $V_{L,0}$ | 2, 0 | mL |
|---|---|---|---|
| Medikamenten-Volumen im Vial 31 | $V_{Med}$, 0 | 5, 0 | mL |
| Flusswiderstand der Auslassleitung 27a | $R_{OUT}$ | 100 | mmHg/ml /min |
| Öffnungsdruck Rückschlagventil 29e | $\Delta P_{,N}$ | 50 | mmHg |
| Öffnungsdruck Rückschlagventil 29a | $\Delta P_{OUT}$ | 50 | mmHg |
| Periodendauer T der Druckoszillationen $$T = V_s \ / \ Q_b \ / \ n$$ mit $V_s$= Schlagvolumen pro Umdrehung; $Q_h$= Blutfluss; n = Rotor-Anzahl | T | 5 | s |

Der Startzeitpunkt beginnt beispielsweise entweder mit dem Einsetzen des Vials 31 in den extrakorporalen Kreislauf 2000, z. B. in ein hierfür vorgesehenes, nicht dargestelltes Vial-Gate, oder alternativ mit dem manuellen Öffnen von Verschlüssen in den Ein- und Auslassleitungen 27e, 27a.

In einer **Füllphase** wird der maximale Druck im Blasenfänger $P_{BF,max}$ festgestellt und/oder gespeichert. Am Ende des Füllzyklus, d. h. wenn der Druck $P_{BF}$ wieder fällt, werden damit folgende Parameter berechnet:

- der im Vial 31 gefangene Druck $P_{Vial,in}$ nach Gleichung (2.4),
- der Startwert $P_{Vial(0)}$ für die Ausströmphase nach Gleichung (2.11),
- die Compliance C nach Gleichung (2.3), und
- die Zeitkonstante $\tau$ nach Gleichung (2.9).

In der **Ausströmphase** werden

- der zeitliche Druckverlauf $P_{Vial}(t)$ im Vial 31 nach Gleichung (2.10); und
- das applizierte Teilvolumen $\Delta V_{Med}$ im aktuellen Zyklus nach Gleichung (2.6) berechnet.

Am Ende der Ausströmphase wird das bisher applizierte Volumen $V_{Med}$ (beispielsweise durch Aufsummieren aller bisherigen Teilvolumina $\Delta V_{Med}$) und die Zeitdauer $t_{ges}$ (beispielsweise mittels Gleichung 2.14) berechnet.

Wenn das bisher applizierte Volumen $V_{Med}$ noch nicht dem insgesamt zu applizierenden Medikamentenvolumen $V_{Med,0}$ entspricht, wird der nächste Zyklus mit der Füllphase gestartet.

Eine verbleibende Restzeit der Applikation kann aus Applikationsdauer $t_{ges}$ abzüglich der bereits vergangenen Zeit, in welcher appliziert wurde, ermittelt werden.

Alternativ kann hierzu die Differenz aus dem insgesamt zu applizierenden Medikamentenvolumen $V_{Med,0}$ und dem bisher applizierten Volumen $V_{Med}$ durch den mittleren Medikamentenfluss $\overline{Q}_{Med}$ geteilt werden.

**Fig. 2** zeigt Drücke $P_{BF}$ und $P_{Vial}$ (jeweils in mmHg) über der Zeit t (in s), welche bei einer exemplarischen Umsetzung der vorliegenden Erfindung aufgetreten sind. Gezeigt sind die Druckverläufe aus zwei Zyklen. Die gezeigten Drücke sind Relativ-Drücke. Die zur Öffnung der Rückschlagventile 29a und 29e benötigten Drücke betrugen jeweils 50 mmHg.

Gezeigt sind neben den Verläufen der Drücke $P_{BF}$ und $P_{Vial}$ auch der Öffnungszustand des Auslassrückschlagventils 29a und der Öffnungszustand des Einlassrückschlagventils 29e.

Die mit 29a bzw. 29e bezeichneten Blöcke der Fig. 2 geben die Zeitdauern an, in welchen das Auslassrückschlagventil 29a bzw. das Einlassrückschlagventil 29e jeweils geöffnet sind. Zu Zeitpunkten, welche nicht in die gezeichneten Blöcke fallen, sind die Ventile jeweils geschlossen.

Der Öffnungszustand eines der Rückschlagventile 29a, 29e ist zwischen etwa t = 1s und t = 2s gezeigt. In dieser Zeitspanne öffnet das Auslassrückschlagventil 29a, und es wird das Medikament aus dem Vial 31 über die Auslassleitung 27a in den Blutschlauchsatz 2000, z. B. in den Blasenfänger 21 hinein, appliziert. Die zwischen Öffnen und Schließen des Auslassrückschlagventils 29a liegende Zeitspanne entspricht einer von zwei in Fig. 2 gezeigten Ausströmphasen. Durch das erstmalige Öffnen des Auslassrückschlagventils 29a steigt auch das in Fig. 2 ebenfalls gezeigte, applizierte Volumen $V_{med}$ an. $V_{med}$ ist als absoluter Wert gezeigt. Das Volumen $V_{med}$ steigt später ab etwa t = 4,5 s erneut an, da zu diesem Zeitpunkt die zweite in Fig. 2 erkennbare Ausströmphase, bedingt durch das erneute Öffnen des Auslassrückschlagventils 29a, beginnt.

Zwischen den beiden vorstehend diskutierten Ausströmphasen liegen zwei Füllphasen, ebenso nach der zweiten Ausströmphase. Die Füllphasen gehen einher mit der Öffnung des Einlassrückschlagventils 29e. Die erste Füllphase beginnt bei etwa t = 2,2 s und endet bei etwa t = 2,5 s. Die zweite Füllphase beginnt bei etwa 3 s und endet bei etwa 3,5 s.

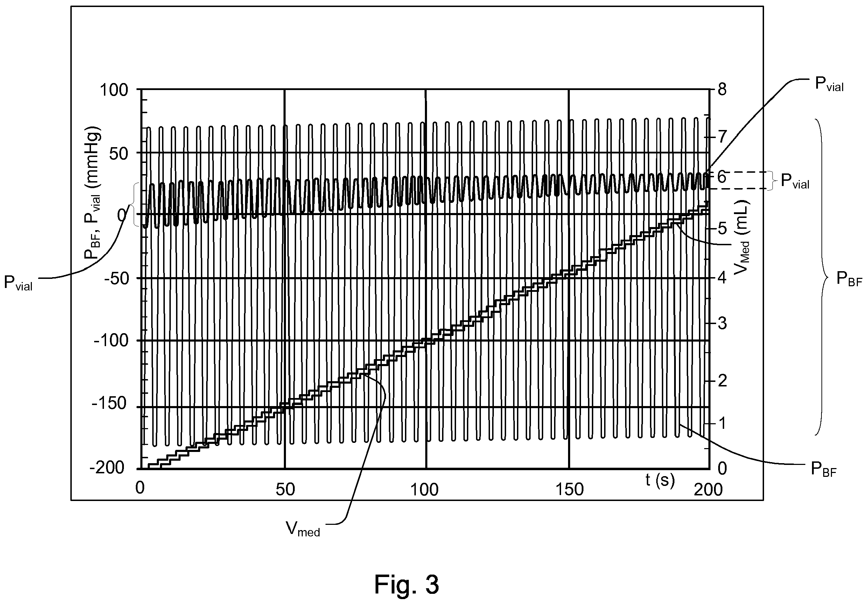

**Fig. 3** zeigt wie Fig. 2 die Drücke $P_{BF}$ und $P_{Vial}$ (jeweils in mmHg) über der Zeit t (in s), welche bei einer exemplarischen Umsetzung der vorliegenden Erfindung aufgetreten sind. Die gezeigten Drücke sind Relativ-Drücke. Die zur Öffnung der Rückschlagventile 29a und 29e benötigten Drücke betrugen jeweils 50 mmHg.

Wie mittels geschweifter Klammern links und rechts neben dem Diagramm angedeutet, schwankt der Druck $P_{Vial}$ bei t = 0 noch zwischen etwa -5 und + 25 mmHg. Diese Amplitude nimmt aber bis t = 200 ab und erstreckt sich dann zwischen etwa 20 und 25 mmHg. Gleichzeitig bleibt die Amplitude des Drucks $P_{BF}$ stabil zwischen etwa -175 und +75 mmHg.

Fig. 3 zeigt ferner das mit der Zeit ansteigende Volumen des aus dem Vial 31 applizierten Medikaments in Lösung. Gut zu erkennen sind die vielen einzelnen Zyklen, in welchen das Medikament jeweils ausgebracht wurde.

In der Praxis wird der der Anwender einen Blutschlauchsatz mit einer Verbindungsvorrichtung 27 zur Aufnahmen eines Vials 31 bereitstellen müssen. Die Verbindungsvorrichtung 27 wird mit einem Vial 31 bestückt.

Ferner wird er in einem entsprechenden Menü die Größe des Vials 31 (vgl. Volumen des Vials: 5 oder 10ml) und die Applikationsrate/Applikationsdauer oder ggf. das Medikament selber auswählen.

Abhängig von der Verfahrensimplementierung kann der Anwender entweder durch Auswahl des entsprechenden Medikaments oder durch Auswahl der Vialgröße und der Applikationsrate (bzw. -dauer) die Dosierung vom Dialysegerät beeinflussen.

Hat der Anwender die Einstellung vorgenommen, wird zunächst der venöse Pegel gesetzt, um ein Benetzen des venösen Transducer Protector durch die Pegelerhöhung (aufgrund der Vialentleerung) am venösen Blasenfänger zu verhindern.

Es kann eine Anzeige z. B. am Monitor des Dialysegeräts eingeblendet sein, um die verbleibende Restzeit der Applikation anzuzeigen, etwa als rückwärts laufende Uhr oder als Stoppuhr.

### Bezugszeichenliste

| Bezugszeichen | Bezeichnung |
| --- | --- |
| 1000 | Funktionseinrichtung |
| 2000 | extrakorporaler Kreislauf |
| 3000 | Blutbehandlungsvorrichtung |
| 4000 | Blutpumpe |
| 5000 | Steuervorrichtung |
| 1 | arterieller Leitungsabschnitt |
| 3 | venöser Leitungsabschnitt |

(fortgesetzt)

| Bezugszeichen | Bezeichnung |
|---|---|
| 9 | arterieller Drucksensor |
| 11 | arterielle Klemme |
| 17 | venöse Klemme |
| 21 | venöser Blasenfänger, Blasenkammer |
| 25 | venöser Drucksensor |
| 27 | Verbindungsvorrichtung |
| 27e | Einlassleitung |
| 27a | Auslassleitung |
| 29e | Einlassrückschlagventil |
| 29a | Auslassrückschlagventil |
| 31 | Vial oder Medikamentenbehälter |
| $P_{BF}$ | im Blasenfänger 21 herrschender Druck |
| Pven | in einem oberen Abschnitt des Blasenfängers 21 herrschender Druck |
| $P_{Vial}$ | im Medikamentenbehälter 31 herrschender Druck |

**Patentansprüche**

1. Blutbehandlungsvorrichtung (3000), aufweisend oder verbunden mit wenigstens einer/einem

   - Pumpe, insbesondere Blutpumpe (4000);
   - Steuereinrichtung (5000);
   - extrakorporalen Kreislauf (2000) mit einem Leitungsinneren, wobei der extrakorporale Kreislauf (2000) verbunden ist mit einer Verbindungsvorrichtung (27) zum Verbinden eines Medikamentenbehälters (31), welcher ein flüssiges Medikament aufweist, mit dem extrakorporalen Kreislauf (2000), wobei die Verbindungsvorrichtung (27) eine Einlassleitung (27e) und eine Auslassleitung (27a) aufweist, jeweils zum Verbinden eines Inneren des Medikamentenbehälters (31) mit dem Leitungsinneren des extrakorporalen Kreislaufs (2000); wobei die Einlassleitung (27e) mit einem ersten Abschnitt des extrakorporalen Kreislaufs (2000) in Fluidkommunikation verbunden ist; und wobei die Auslassleitung (27a) mit einem zweiten Abschnitt des extrakorporalen Kreislaufs (2000) in Fluidkommunikation verbunden ist; und wobei der extrakorporale Kreislauf (2000) verbunden ist mit der Blutbehandlungsvorrichtung (3000);
   - Drucksensor (25) zum Ermitteln des Drucks im ersten oder zweiten Abschnitt;

   **dadurch gekennzeichnet, dass** die Steuervorrichtung (5000) konfiguriert und/oder programmiert ist zum

   - Bestimmen eines Parameters, welcher eine Menge oder ein Volumen eines bereits applizierten Medikaments und/oder eine zur vollständigen Verabreichung einer vorbestimmten Menge oder eines vorbestimmten Volumens des Medikaments erforderliche Restzeit ist, wobei der im Medikamentenbehälter (31) herrschende Druck ermittelt oder mittels Modells berechnet wird,

   und dadurch, dass die Einlassleitung (27e) ein erstes Rückschlagventil (29e), welches ab einem Öffnungsdruck ($\Delta P_{IN}$) für dieses Ventil öffnet, aufweist, welches einen Fluss nur in Richtung zum Inneren des Medikamentenbehälters (31) erlaubt; wobei die Auslassleitung (27a) ein zweites Rückschlagventil (29a), welches ab einem Öffnungsdruck ($\Delta P_{OUT}$) für dieses Ventil öffnet, aufweist, welches einen Fluss nur in Richtung zum Äußeren des Medikamentenbehälters (31) erlaubt.

2. Blutbehandlungsvorrichtung (3000) nach Anspruch 1, wobei die Steuervorrichtung (5000) ferner konfiguriert und/oder programmiert ist zum

- Ermitteln eines während eines Pumpzyklusses der Pumpe oder der Blutpumpe (4000) über die Auslassleitung (27a) in den extrakorporalen Kreislauf (2000) abgegebenen Teilvolumens ($\Delta V_{Med}$) des flüssigen Medikaments.

**3.** Blutbehandlungsvorrichtung (3000) nach einem der Ansprüche 1 bis 2, wobei die Steuervorrichtung (5000) ferner konfiguriert und/oder programmiert ist zum

- Ermitteln eines über bisherige Pumpzyklen der Pumpe oder der Blutpumpe (4000) summiert aus dem Medikamentenbehälter (31) in den extrakorporalen Kreislauf (2000) abgegebenen Volumens ($V_{Med}$) des flüssigen Medikaments.

**4.** Blutbehandlungsvorrichtung (3000) nach einem der Ansprüche 1 bis 3, wobei die Steuervorrichtung (5000) ferner konfiguriert und/oder programmiert ist zum

- Ermitteln der Differenz zwischen eines insgesamt zur Abgabe in den extrakorporalen Kreislauf (2000) abzugebenden Volumens des Medikaments und des summiert in den extrakorporalen Kreislauf (2000) abgegebenen Volumens ($V_{Med}$); und optional
- Ermitteln und Anzeigen einer bis zum Erreichen des abzugebenden Volumens erforderlichen Restzeit.

**5.** Blutbehandlungsvorrichtung (3000) nach einem der Ansprüche 1 bis 4, wobei die Steuervorrichtung (5000) ferner konfiguriert und/oder programmiert ist zum

- Ermitteln einer Zeitdauer ($t_{OUT}$) während welcher das Rückschlagventil (29a) der Auslassleitung (27a) geöffnet ist oder war.

**6.** Blutbehandlungsvorrichtung (3000) nach einem der Ansprüche 1 bis 5, wobei die Steuervorrichtung (5000) ferner konfiguriert und/oder programmiert ist zum

- Ermitteln des im ersten und/oder zweiten Abschnitt herrschenden Drucks ($P_{BF}$) während wenigstens eines Pumpzyklus der Pumpe oder der Blutpumpe (4000) oder während eines vorbestimmten Zeitraums, vorzugsweise mittels des Drucksensors (25);
- Speichern eines maximalen Wertes ($P_{BFmax}$) des ermittelten Drucks ($P_{BF}$).

**7.** Blutbehandlungsvorrichtung (3000) nach einem der Ansprüche 1 bis 6, wobei die Steuervorrichtung (5000) ferner konfiguriert und/oder programmiert ist zum

- Einbringen von Luft in das Innere des extrakorporalen Kreislaufs (2000), insbesondere in den venösen Blasenfänger (21) hiervon.

**8.** Blutbehandlungsvorrichtung (3000) nach einem der Ansprüche 1 bis 7, ausgestaltet als Hämodialysevorrichtung, Hämofiltrationsvorrichtung oder Hämodiafiltrationsvorrichtung.


**Claims**

**1.** A blood treatment apparatus (3000), comprising or connected to at least one

- pump, in particular blood pump (4000);
- control device (5000);
- extracorporeal circuit (2000) having a line interior, wherein the extracorporeal circuit (2000) is connected to a connecting device (27) for connecting a drug container (31) comprising a liquid drug to the extracorporeal circuit (2000), wherein the connecting device (27) comprises an inlet line (27e) and an outlet line (27a), to connect, respectively, an interior of the drug container (31) to the line interior of the extracorporeal circuit (2000); wherein the inlet line (27e) is connected in fluid communication to a first section of the extracorporeal circuit (2000); and wherein the outlet line (27a) is connected in fluid communication to a second section of the extracorporeal circuit (2000) ; and wherein the extracorporeal circuit (2000) is connected to the blood treatment apparatus (3000); 
- pressure sensor (25) for determining the pressure in the first or second section;

**characterized in that** the control device (5000) is configured and/or programmed for

- determining a parameter, which is an amount or a volume of an already applied drug and/or a remaining time required for the complete delivery of a predetermined amount or a predetermined volume of drug, the pressure prevailing in the drug container (31) being determined or calculated by means of a model,

and **in that** the inlet line (27e) comprises a first check valve (29e) which opens from an opening pressure ($\Delta P_{IN}$) for this valve, which allows a flow only towards the interior of the drug container (31); wherein the outlet line (27a) comprises a second check valve (29a) which opens from an opening pressure ($\Delta P_{OUT}$) for this valve, which allows a flow only towards the exterior of the drug container (31).

2. The blood treatment apparatus (3000) according to claim 1, wherein the control device (5000) is further configured and/or programmed for

- determining a part-volume ($\Delta V_{Med}$) of the liquid drug delivered through the outlet line (27a) into the extracorporeal circuit (2000) during a pump cycle of the pump or of the blood pump (4000).

3. The blood treatment apparatus (3000) according to anyone of claims 1 to 2, wherein the control device (5000) is further configured and/or programmed for

- determining a total volume ($V_{Med}$) of the liquid drug delivered from the drug container (31) into the extracorporeal circuit (2000) during preceding pump cycles of the pump or of the blood pump (4000).

4. The blood treatment apparatus (3000) according to anyone of claims 1 to 3, wherein the control device (5000) is further configured and/or programmed for

- determining the difference between a total volume of the drug to be delivered into the extracorporeal circuit (2000) and the total volume ($V_{Med}$) delivered into the extracorporeal circuit (2000); and optionally
- determining and displaying a remaining time required for reaching the volume intended to be delivered.

5. The blood treatment apparatus (3000) according to anyone of claims 1 to 4, wherein the control device (5000) is further configured and/or programmed for

- determining a period of time ($t_{OUT}$) during which the check valve (29a) of the outlet line (27a) is open or has been open.

6. The blood treatment apparatus (3000) according to anyone of claims 1 to 5, wherein the control device (5000) is further configured and/or programmed for

- determining, preferably by means of the pressure sensor (25), the pressure ($P_{BF}$) prevailing in the first and/or in the second section during at least one pump cycle of the pump or of the blood pump (4000), or during a predetermined period of time;
- storing a maximum value ($P_{BFmax}$) of the detected pressure ($P_{BF}$).

7. The blood treatment apparatus (3000) according to anyone of claims 1 to 6, wherein the control device (5000) is further configured and/or programmed for

- introducing air into the interior of the extracorporeal circuit (2000), in particular into the venous bubble trap (21) thereof.

8. The blood treatment apparatus (3000) according to anyone of claims 1 to 7, designed as a hemodialysis apparatus, hemofiltration apparatus or hemodiafiltration apparatus.


**Revendications**

1. Un appareil de traitement du sang (3000) comprenant ou étant relié à au moins

- une pompe, notamment une pompe à sang (4000);
- un dispositif de commande (5000);

- un circuit extracorporel (2000) ayant un intérieur de conduit, où le circuit extracorporel (2000) est relié à un dispositif de liaison (27) pour relier un récipient à médicament (31) comprenant un médicament liquide au circuit extracorporel (2000), où le dispositif de liaison (27) comprend un conduit d'entrée (27e) ainsi qu'un conduit de sortie (27a), pour relier, respectivement, un intérieur du récipient à médicament (31) à l'intérieur de conduit du circuit extracorporel (2000); où le conduit d'entrée (27e) est relié en communication fluidique à une première partie du circuit extracorporel (2000); et où le conduit de sortie (27a) est relié en communication fluidique à une seconde partie du circuit extracorporel (2000); et où le circuit extracorporel (2000) est relié à l'appareil de traitement du sang (3000);
- un capteur de pression (25) pour déterminer la pression dans la première ou dans la seconde partie;

**caractérisé en ce que** le dispositif de commande (5000) est configuré et/ou programmé pour

- déterminer un paramètre, qui est une quantité ou un volume d'un médicament déjà appliqué et/ou un temps résiduel requis pour l'administration complète d'une quantité ou d'un volume prédéterminé(e) du médicament, la pression régnant dans le récipient à médicament (31) étant déterminée ou calculée au moyen d'un modèle,

et **en ce que** le conduit d'entrée (27e) comporte un premier clapet anti-retour (29e) qui s'ouvre à partir d'une pression d'ouverture ($\Delta P_{IN}$) de ce clapet, qui ne permet un écoulement que vers l'intérieur du récipient à médicament (31); où le conduit de sortie (27a) comporte un second clapet anti-retour (29a) qui s'ouvre à partir d'une pression d'ouverture ($\Delta P_{OUT}$) de ce clapet, qui ne permet un écoulement que vers l'extérieur du récipient à médicament (31).

2. L'appareil de traitement du sang (3000) selon la première revendication, où le dispositif de commande (5000) est en outre configuré et/ou programmé pour

   - déterminer un volume partiel ($\Delta V_{Med}$) du médicament liquide administré via le conduit de sortie (27a) dans le circuit extracorporel (2000) pendant un cycle de pompage de la pompe ou de la pompe à sang (4000).

3. L'appareil de traitement du sang (3000) selon l'une quelconque des revendications 1 à 2, où le dispositif de commande (5000) est en outre configuré et/ou programmé pour

   - déterminer un volume ($V_{Med}$) total du médicament liquide administré depuis le récipient à médicament (31) dans le circuit extracorporel circuit (2000) pendant les cycles de pompage précédents de la pompe ou de la pompe à sang (4000) .

4. L'appareil de traitement du sang (3000) selon l'une quelconque des revendications 1 à 3, où le dispositif de commande (5000) est en outre configuré et/ou programmé pour

   - déterminer la différence entre un volume total du médicament à administrer dans le circuit extracorporel (2000) et le volume ($V_{Med}$) total administré dans le circuit extracorporel (2000); et facultativement
   - déterminer et afficher un temps résiduel requis pour atteindre le volume à administrer.

5. L'appareil de traitement du sang (3000) selon l'une quelconque des revendications 1 à 4, où le dispositif de commande (5000) est en outre configuré et/ou programmé pour

   - déterminer une période de temps ($t_{OUT}$) pendant laquelle le clapet anti-retour (29a) du conduit d'entrée (27a) est ou a été ouvert.

6. L'appareil de traitement du sang (3000) selon l'une quelconque des revendications 1 à 5, où le dispositif de commande (5000) est en outre configuré et/ou programmé pour

   - déterminer, de préférence au moyen du capteur de pression (25), la pression ($P_{BF}$) régnant dans la première et/ou dans la seconde partie durant au moins un cycle de pompage de la pompe ou de la pompe à sang (4000), ou durant une période de temps prédéterminée;
   - enregistrer une valeur maximale ($P_{BFmax}$) de la pression détectée ($P_{BF}$).

7. L'appareil de traitement du sang (3000) selon l'une quelconque des revendications 1 à 6, où le dispositif de commande (5000) est en outre configuré et/ou programmé pour

- introduire de l'air à l'intérieur du circuit extracorporel (2000), notamment dans le piège à bulles veineux (21) de celui-ci.

8. L'appareil de traitement du sang (3000) selon l'une quelconque des revendications 1 à 7, conçu comme un appareil d'hémodialyse, appareil d'hémofiltration ou appareil d'hémodiafiltration.

Fig. 1

Fig. 2

EP 3 386 560 B1

Fig. 3

**EP 3 386 560 B1**

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- EP 2319553 A1
- US 20140039447 A1
- US 20130218123 A1
- US 20120150141 A1
- US 20050145009 A1
- US 20110257591 A1
- DE 102007026010 A1
- DE 102009018664 A1
- DE 102009024468 A1